(19) Europäisches Patentamt
European Patent Office
Office européen des brevets

(11) EP 4 786 589 A1

(12) EUROPEAN PATENT APPLICATION
published in accordance with Art. 153(4) EPC

(43) Date of publication:
**05.08.2026 Bulletin 2026/32**

(21) Application number: **24873041.8**

(22) Date of filing: **27.09.2024**

(51) International Patent Classification (IPC):
***C12N 15/11*** (2006.01) ***C12N 15/113*** (2010.01)
***C12N 15/63*** (2006.01)

(52) Cooperative Patent Classification (CPC):
**C12N 15/11; C12N 15/113; C12N 15/63**

(86) International application number:
**PCT/KR2024/014734**

(87) International publication number:
**WO 2025/071334 (03.04.2025 Gazette 2025/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.09.2023 KR 20230130727**

(71) Applicant: **Hanmi Pharm. Co., Ltd.**
**Hwaseong-si, Gyeonggi-do 18536 (KR)**

(72) Inventors:
- **KIM, Inn Ah**
  **Hwaseong-si, Gyeonggi-do 18469 (KR)**
- **SHIN, Seung Hyun**
  **Hwaseong-si, Gyeonggi-do 18469 (KR)**
- **LEE, Ji Hee**
  **Hwaseong-si, Gyeonggi-do 18469 (KR)**
- **LIM, Chang Gyu**
  **Hwaseong-si, Gyeonggi-do 18469 (KR)**
- **HAN, Seung Su**
  **Hwaseong-si, Gyeonggi-do 18469 (KR)**
- **HAN, Young Jin**
  **Hwaseong-si, Gyeonggi-do 18469 (KR)**
- **HEO, Yong Ho**
  **Hwaseong-si, Gyeonggi-do 18469 (KR)**

(74) Representative: **Santarelli**
**Tour Trinity**
**1 bis Place de la Défense**
**92400 Courbevoie (FR)**

Remarks:
The complete document including Reference Table(s) and the Sequence Listing(s) can be downloaded from the EPO website

# (54) NON-NATURAL 3' UNTRANSLATED REGION AND USE THEREOF

(57) Provided are a messenger RNA (mRNA) including a non-natural 3' untranslated region (3'UTR) and having excellent activity of increasing a synthesis yield of the mRNA of a gene, a composition including the mRNA, a composition for delivering the mRNA or a polypeptide to a subject including the mRNA, and DNA encoding the mRNA.

EP 4 786 589 A1

## Description

### Technical Field

The present disclosure relates to a non-natural 3' untranslated region and use thereof.

### Background Art

Messenger RNA (mRNA) is frequently used as an alternative to plasmid DNA as a gene delivery molecule in the field of cancer immunotherapy and stem cell-based biomedical research. As a direct source of gene products, mRNA has several advantages including the lack of a requirement for nuclear entry, which poses a significant barrier to DNA delivery, especially in non-dividing cells. Also, by avoiding the expression of oncogenes caused by abnormal transcription and insertional mutagenesis, mRNA is less likely to integrate into the host genome.

For a given gene, untranslated regions (UTRs) of the gene including a 5'UTR and a 3'UTR are regions involved in regulating expression. The 5'UTR is a DNA regulatory region at the 5' end of all sequences encoding a protein, and is transcribed into mRNA but is not translated into a protein. The 5'UTR may include various regulatory elements, such as a 5'-cap structure, a G-quadruplex structure (G4), a stem-loop structure, and an internal ribosome entry site (IRES), and such regulatory elements play an important role in controlling translation initiation. The 3'UTR positioned downstream of the sequence encoding a protein is known to be involved in many regulatory processes including transcript cleavage, stability and polyadenylation, translation, and mRNA localization. The 3'UTR serves as a binding site for many regulatory proteins and small non-coding RNAs (e.g., microRNAs).

In general, current mRNA therapies are often not tissue-specific or rely on native or standard UTR sequences that provide a low level of protein expression. In this regard, there is a need for novel 3'UTRs and a combination thereof that can stabilize mRNA therapies and increase protein synthesis.

### Disclosure of Invention

### Technical Problem

An aspect provides a messenger RNA (mRNA) including a non-natural 3' untranslated region (3'UTR).

Another aspect provides DNA encoding the mRNA.

Another aspect provides a method of obtaining the mRNA, the method including transcribing the mRNA by using the DNA as a template.

Another aspect provides a method of obtaining a polypeptide, the method including translating the mRNA.

Another aspect provides a composition including the mRNA.

Another aspect provides a method of utilizing the mRNA, the method including introducing the mRNA to a host cell.

### Solution to Problem

As used in the present specification, "5' untranslated region (5'-UTR)" refers to an mRNA region where a polypeptide is not encoded, directly upstream (i.e., 5') of the first codon, i.e., an initiation codon, of an mRNA transcript to be translated by ribosomes.

As used in the present specification, "3' untranslated region (3'-UTR)" refers to an mRNA region where a polypeptide is not encoded, directly downstream (i.e., 3') of a stop codon, i.e., a termination codon, of an mRNA transcript signaling termination of translation.

As used in the present specification, "open reading frame (ORF)" or "sequence encoding a polypeptide" refers to a continuous region of DNA that starts with an initiation codon, such as a methionine codon (ATG) and ends with a stop codon, such as TAA, TAG, or TGA, and encodes a polypeptide.

As used in the present specification, "polyadenylate sequence," "poly (A)," or "poly (A) tail" refers to an mRNA region including multiple continuous adenine nucleotides, downstream of the 3'-UTR, for example, directly downstream (i.e., 3') of the 3'-UTR. The poly (A) tail may include 1,000, 500, 400, or 300 or less adenine nucleotides. For example, the poly (A) tail may include 10 to 300 adenine nucleotides. For example, the poly (A) tail may include 10, 20, 30, 40, 50, 60, 70, 80, 90, 100, 110, 120, 130, 140, 150, 160, 170, 190, 200, 210, 220, 230, 240, 250, 260, 270, 280, 290, or 300 nucleotides. The poly (A) tail may include, for example, 50 to 250 adenine nucleotides. The poly (A) tail in a living body, such as a cell or a subject, may serve to protect the mRNA from an attack of an enzyme, such as a cytoplasmic enzyme, and aid in termination of transcription, export of the mRNA from the nucleus, and translation. A 3' poly (A) tail refers to a region (stretch) of the adenine nucleotides, and is generally added to mRNA that is transcribed during mRNA processing or to immature mRNA.

As used in the present specification, the term "operatively linked" refers to connection of nucleotide sequences on a single nucleic acid fragment so that a function is affected by another. For example, a promoter is operatively linked to a coding sequence (e.g., an ORF) when the promoter is capable of affecting the expression of the coding sequence (that is, when transcription of the coding sequence is regulated by the promoter). The coding sequence may be operatively linked to a regulatory sequence in a sense direction or an anti-sense direction. Unless otherwise stated in the present specification, each nucleic acid sequence included in the claimed mRNA or DNA is operatively linked to one another.

As used herein, unless a location of a nucleotide sequence is otherwise specified, a nucleotide sequence is connected or located from the 5'-end to the 3'-end.

As used in the present specification, the term "vector" or "nucleic acid construct" refers to any nucleic acid capable of carrying a gene, an ORF, or a DNA fragment into a cell. The vector may be, for example, one which may be replicated in a cell. The vector may be a virus, a bacteriophage, a provirus, a plasmid, a phagemid, a transposon, or an artificial chromosome such as an yeast artificial chromosome (YAC), a bacterial artificial chromosome (BAC), a plant artificial chromosome (PLAC), etc.

The first aspect provides an mRNA including a non-natural 3' untranslated region (3'UTR),

wherein the 3'UTR has: a secondary structure represented by dot-bracket notation below:

.

(((((...........(((...(((((.(((....))).)))))...)))..(((((((..((((....((((((.(((((.......)))))..))-
).)))))))...)))) ))).......(((....)))............(((.........(((....))))))..)))); or
a secondary structure represented by dot-bracket notation below:
(((((((((((...(((((.((........((((((((.........))))))))))))))).....(((...))).....)))))))))))..((((((((((((....) )))))...)))))).....((((((...))))))...........,
wherein a dot denotes unpaired bases in a sequence, and "(" and ")" denote paired bases in a sequence. As used in the present specification, the term "secondary structure" refers to binding between paired bases.

The mRNA including the 3'UTR may have a mean free energy of -0.1 to -0.3 kcal/mol for RNA folding,

(a) the secondary structure of the mRNA comprising the 3'UTR, represented by

.

(((((...........(((...(((((.(((....))).)))))...)))..(((((((..((((....((((((.(((((.......)))))..))-
).)))))))...)))) ))).......(((....)))............(((.........(((....))))))..))))), has a length of 178 nt and a GC-content (GC %) of 40 to 50 %, and
(b) the secondary structure of the mRNA comprising the 3'UTR, represented by
(((((((((((...(((((.((........((((((((.........))))))))))))))).....(((...))).....)))))))))))..((((((((((((....) )))))...)))))).....((((((...))))))...........,
has a length of 158 nt and a GC% of 30 to 50 %.

The mRNA including the 3'UTR may include a sequence selected from the group consisting of nucleotide sequences of SEQ ID NOs: 1 to 8.

The 3'UTR having the secondary structure of (a) may include a nucleotide sequence of any one of SEQ ID NOs: 1 to 5. The 3'UTR having the secondary structure of (b) may include a nucleotide sequence of any one of SEQ ID NOs: 6 to 8.

The mRNA may consist of a nucleotide sequence of any one of SEQ ID NOs: 1 to 8. The mRNA may be an isolated nucleic acid, e.g., a transcript, including a nucleotide sequence of any one of SEQ ID NOs: 1 to 8. The transcript may include a sequence encoding a target protein.

The mRNA may further include a sequence encoding a polypeptide. The sequence encoding a polypeptide may be operatively linked to the upstream of the 3'UTR.

The polypeptide may be an antigenic or therapeutic polypeptide. The antigenic polypeptide may be a viral antigenic polypeptide.

The therapeutic polypeptide may be any polypeptide having disease-treating activity, for example, anticancer activity. The polypeptide having anticancer activity may be a tumor suppressor, an immune stimulating protein, a radiation enhancing protein, or a chemotherapy enhancing protein. For example, the tumor suppressor may be involved in regulating the cell cycle of cancer cells themselves, and may accordingly have an anticancer activity. Immune cells involved in the immune stimulation may include B cells, NK cells, T cells, or mast cells. The polypeptide having anticancer activity may not have antigenicity in a host cell or an organism including the same. The polypeptide having anticancer activity may be an antibody, a hormone, a cytokine, an enzyme, a derivative thereof, or a combination thereof. Also, the polypeptide having anticancer activity may suppress proliferation of cancer cells. The polypeptide may be a recombinant one including a fusion protein.

The sequence encoding a polypeptide may be codon-optimized. The codon optimization may vary depending on a host cell to which the mRNA molecule is introduced. Also, the sequence encoding a polypeptide may include a degenerate sequence.

The mRNA may further include an expression regulatory sequence. The expression regulatory sequence may be involved in translational regulation or mRNA stability. The expression regulatory sequence may be a Kozak sequence, a sequence encoding the 5'UTR, or a poly (A) sequence.

The sequence encoding the 3'UTR, the sequence encoding a polypeptide, and the expression regulatory sequence may be transcribed *in vitro* or *in vivo*, and may be form a common transcript.

The sequence encoding the 5'UTR may be operatively linked to the 5'UTR upstream of the sequence encoding a polypeptide.

The sequence encoding the 5'UTR may be derived from any gene. When the 5'UTR is combined with the 3'UTR, expression efficiency of RNA may be synergistically improved.

The poly (A) sequence may be operatively linked to the 3'UTR downstream of the 3'UTR.

The 3'UTR may have activity of increasing the translation yield, stability of the RNA, or a combination thereof, compared to a case where the native 3'UTR is used.

In an embodiment, the mRNA may include "5'-cap" such as Cap0, Cap1, or Cap2. The term "5'-cap" refers to a cap structure found at the 5'-end of an mRNA molecule. The 5'-cap is generally a 7-methylguanine ribonucleotide connected to the mRNA via 5' triphosphate at the 5' position of the first nucleotide in a 5'-to-3' chain. In the cap0, riboses at the first and second cap-proximal nucleotides of the mRNA may both include 2'-hydroxyl. In the cap1, riboses at the first and second cap-proximal nucleotides of the mRNA may include 2'-methoxy and 2'-hydroxyl, respectively. In the cap2, riboses at the first and second cap-proximal nucleotides of the mRNA may both include 2'-methoxy. The 7-methylguanine ribonucleotide may be further modified. For example, in the modified 7-methylguanine ribonucleotide, 7-methylguanine 3'-methoxy (7mG(3'OMe)) or 2'-hydroxyl and 3'-hydroxyl may each independently be substituted with a sulfonyl-containing group selected from the group consisting of a mesyl group, an esyl group, a triflyl group, a tresyl group, a tosyl group, a brosyl group, a nosyl group, and a dansyl group. The modified 7-methylguanine ribonucleotide substituted with the sulfonyl-containing group may be, for example, 7-methylguanine 3'-mesyl (7mG(3'OMs)). Most endogenous mRNAs in higher eukaryotes including mammalian mRNA such as human mRNA may have the cap1 or the cap2. The Cap0 and other cap structures different from Cap1 and Cap2 may be immunogenic in mammals such as humans.

Such caps may be introduced co-transcriptionally. For example, the 5'-cap or a 5'-cap analog may be introduced to a transcript at initiation at which the 5'-cap or the 5'-cap analog is generated, while a DNA template is transcribed *in vitro* in the presence of the 5'-cap or the 5'-cap analog.

Also, such caps may be introduced post-transcriptionally. The 5'-cap or the 5'-cap analog may be introduced to RNA post-transcriptionally by a capping enzyme such as a capping enzyme from vaccinia virus. The 5'-end sequence with the 5'-end cap structure formed may include, for example, 7mG(5')ppp(5')ApG, m7G(3'OMe)(5')ppp(5')ApG, m7G(3'OMe)(5')ppp(5')(2'OMeA)pG, m7G (3'OMs)(5')ppp(5')(2'OMeA)pG, or m7G(5')ppp(5')(2'OMeA)pG.

In an embodiment, the mRNA may include a modified nucleotide. In an embodiment, the mRNA may have at least one U substituted with N1-methyl-pseudouridine. In the mRNA, the 5'-end sequence with the 5'-end cap structure formed may have a cap structure of m7(3'OMeG)(5')ppp(5')(2'OMeA)pG or a cap structure of m7(3'OMsG)(5')ppp(5')(2'OMeA)pG. In the mRNA, all Us may be substituted with N1-methyl-pseudouridine. In the mRNA, all Us may be substituted with N1-methyl-pseudouridine, and the 5'-end sequence with the 5'-end cap structure formed may have a cap structure of m7(3'OMeG)(5')ppp(5')(2'OMeA)pG or a cap structure of m7(3'OMsG)(5')ppp(5')(2'OMeA)pG.

The mRNA may include a nucleotide sequence of any one of SEQ ID NOs: 1 to 8, wherein at least one U of the sequence may be substituted with N1-methyl-pseudouridine. In the mRNA, all Us of the sequence may be substituted with N1-methyl-pseudouridine.

The second aspect provides a method of obtaining a polypeptide, the method including translating the mRNA including the sequence encoding a polypeptide.

The third aspect provides a composition including the mRNA as an active ingredient. The composition may be for delivering the mRNA or a product expressed thereby to a subject.

The fourth aspect provides a composition including the mRNA as an active ingredient, the mRNA including the mRNA or a product expressed thereby to induce a therapeutic response or an immune response to the polypeptide.

In the third aspect and the fourth aspect, the composition may be for providing immunogenicity or for treating a disease. The disease may differ according to the selected polypeptide. The disease may be, for example, infection caused by a virus or a foreign cell, a cancer, a metabolic disease, an inflammatory disease, a gastrointestinal disease, an endocrine disease, sepsis, or an autoimmune disease. In the disease, the foreign cells may be prokaryotic or eukaryotic cells. The foreign cell may be bacteria.

The mRNA may include a sequence encoding a polypeptide. The polypeptide may be an antigenic or therapeutic polypeptide. The antigenic polypeptide may be a viral antigenic polypeptide. The polypeptide is as described in the first aspect.

Also, the mRNA may have anticancer activity by regulating the expression of other genes such as siRNA or miRNA.

The composition may be brought in contact with a cell, a tissue, or a subject in an "effective amount" of the mRNA.

The effective amount may be determined based on a target tissue, a target cell, a means of administration, physical properties of the mRNA (e.g., size of the mRNA and an amount of modified nucleoside), and other components of the composition. Generally, the effective amount of the composition may cause an induced or boosted immune response as a function of the antigen production in cells.

The composition may be administered by intramuscular, subcutaneous, intradermal, intranasal, or pulmonary administration.

The composition may include at least one pharmaceutically acceptable carrier or an excipient. In the composition, the mRNA may be formulated with or in complex with the carrier or the excipient. The carrier or the excipient may be one known in the art.

Relative amounts of active ingredients, pharmaceutically acceptable carriers or excipients, and/or other additional components included in the composition may vary depending on the identity, size and/or conditions of the subject to be treated, and/or routes of administration. For example, the composition may include 0.1 % to 100 %, for example, 0.5 % to 50 %, 1.0 % to 30 %, 5.0 % to 80 %, or 80 %(w/w) or more of active ingredients.

The composition may be formulated as nanoparticles. The nanoparticles may be those known in the art for delivering a polynucleotide, such as an mRNA, into a cell. For example, the nanoparticles may be those known to be used in an RNA vaccine. The nanoparticles may be lipid nanoparticles (LNPs). The composition may be formulated as or bound to LNPs. The binding may include binding with LNPs or binding to the surface of LNPs. For example, the composition may be formulated inside a lipid polycation complex, or may be bound to a lipid polycation complex. The lipid polycation complex is also known as a cationic lipid nanoparticle. The polycation may include MC3, Lipid 319, C12-200, 5A2-SC8, 306Oi10, Moderna Lipid 5, Acuitas A9, SM-102, ALC-0315, Arcturus Lipid 2,2 (8,8) 4C CH3, Genevant CL1, or cationic polypeptides such as polylysine, polyornithine, and/or polyarginine. Also, the composition may be formulated with or bound to LNPs including 1,2 distearoyl-sn-glycero-3-phosphocholine (DSPC), dipalmitoylphosphatidylcholine (DPPC), sterol such as cholesterol, or a non-cationic lipid such as dioleoylphosphatidylethanolamine (DOPE). Also, the composition may be formulated with or bound to LNPs including a polyethyleneglycol (PEG)-lipid such as 1,2-dimyristoyl-rac-glycero-3-methoxypolyethylene glycol-2000 (PEG2000-DMG), 2-[(polyethylene glycol)-2000]-N,N-ditetradecylacetamide (ALC-0159), and polyethylene glycoldimethacrylate (PEG-DMA).

The LNP formulation may include cationic lipids, phospholipids, sterols such as cholesterol, PEG-lipids, or a combination thereof. The LNP formulation may include, for example, cationic lipids, phospholipids, sterols such as cholesterol, and PEG-lipids. Also, the LNPs may include PEG-modified lipids, non-cationic lipids, sterols, ionizable lipids, or a combination thereof. The LNPs may include 0.5 to 15 mol% of PEG-modified lipids, 5 to 25 mol% of non-cationic lipids, 25 to 55 mol% of sterols, and 20 to 60 mol% of ionizable lipids. The PEG-modified lipid may be PEG2000-DMG, the non-cationic lipid may be DSPC, the sterol may be cholesterol, and the ionizable cationic lipid may be Compound 1 having the following structure:

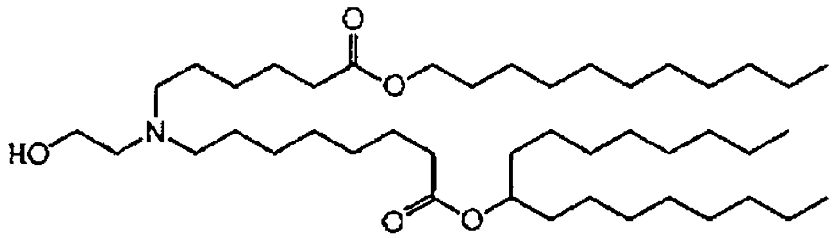

(Compound 1).

The PEG-modified lipid may be Compound 2 (ALC-0159) having the following structure, the non-cationic lipid may be DSPC, the sterol may be cholesterol, and the ionizable cationic lipid may be Compound 3 (ALC-0315) having the following structure:

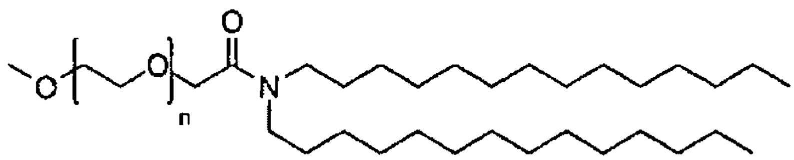

(Compound 2); and

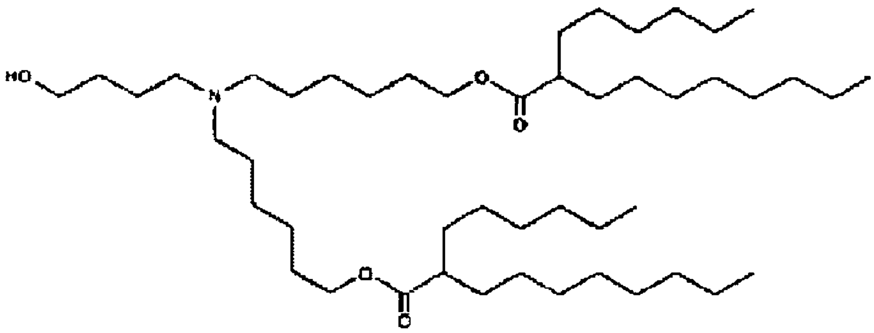

(Compound 3).

The fifth aspect provides a method of utilizing the mRNA, the method including introducing the mRNA to a host cell.

In the method, the host cell may be any cell derived from a subject. The subject may be a mammal including a human. The cell may be, for example, a stem cell, a reproductive cell or a somatic cell. In the method, the host cell may be an antigen-presenting cell including a dendritic cell, a monocyte, or a macrophage.

The method may include administering the mRNA into a subject. The administering may be parenteral or oral administration. The administering may be intramuscular, subcutaneous, intradermal, intranasal, or pulmonary administration. The subject may be a mammal including a human.

The method may be to immunize the subject against the polypeptide or to induce a therapeutic response to the therapeutic polypeptide. In addition, the method may be to treat a disease in a subject.

The sixth aspect provides DNA encoding the mRNA.

The DNA may further include a sequence for introducing a transcribable nucleotide sequence. The sequence for introducing a transcribable nucleotide sequence may be operatively linked to a 3'UTR upstream of a nucleotide encoding the 3'UTR. The sequence for introducing a transcribable nucleotide sequence may be a cloning site. The cloning site may be a multiple cloning site. The cloning site may include a recognition site for a restriction enzyme, a cleavage site, or a combination thereof.

The DNA may further include a promoter sequence. The promoter may be operatively linked to a 5'UTR upstream of a nucleotide encoding the 5'UTR.

The promoter, the nucleotide sequence encoding the 5'-UTR, the sequence encoding a polypeptide or the sequence for introducing a transcribable nucleotide, and the nucleotide sequence encoding the 3'UTR may be linked to one another to form a common transcript when transcription occurs *in vitro* or *in vivo*.

The DNA may be a vector. The vector may be an expression vector or a transcription vector, for the expression of a target protein or an mRNA.

The seventh aspect provides a method of obtaining RNA, the method including transcribing RNA by using the DNA as a template. The transcribing may include in vitro, ex vivo, or in vivo transcription. The in vivo transcription may include transcription occurring in a subject.

The term "in vitro transcription" or "RNA in vitro transcription" refers to a process of synthesizing RNA including an mRNA in a non-cell system, that is, in vitro. The cloning vector DNA including a plasmid DNA vector may be applied as a template for generating an RNA transcript. These cloning vectors are also generally known as transcription vectors. RNA may be obtained by DNA-dependent in vitro transcription with a suitable DNA template. The DNA template may be a linearized plasmid DNA template. The promoter controlling RNA in vitro transcription may be any promoter for a DNA-dependent RNA polymerase. The DNA-dependent RNA polymerase may be a T7 RNA polymerase, a T3 RNA polymerase, a SP6 RNA polymerase, or a combination thereof. The DNA template for RNA in vitro transcription may be obtained by cloning a nucleic acid and introducing the same to a vector for RNA in vitro transcription. The DNA may include cDNA corresponding to each RNA to be transcribed in vitro. The vector for RNA in vitro transcription may be circular plasmid DNA. The cDNA may be obtained by reverse-transcription of an mRNA or by chemical synthesis.

The transcription vector may include a promoter, a nucleotide sequence encoding the 5'-UTR, a sequence encoding a polypeptide, such as an ORF, a nucleotide sequence encoding the 3'-UTR, and a poly (A) tail. The nucleotide sequence encoding the 3'-UTR may include at least one of nucleotide sequences of SEQ ID NOs: 1 to 8. The nucleotide sequence encoding the 5'UTR may include any known 5'UTR sequence. The transcription vector may also include a replication origin, such as *E. coli* replication origin ColE1 ori, a selection marker gene, or a combination thereof. The promoter may be a T7 promoter. The selection marker may be an antibiotic resistance enzyme, such as a kanamycin resistance enzyme.

The in vitro transcription may include obtaining a transformed host cell by introducing the transcription vector to a host cell, for example, *E. coli*, and proliferating the host cell, which includes the plasmid DNA that becomes a template for

an mRNA, by culturing the host cell. The in vitro transcription may include isolating the plasmid DNA from the proliferated host cell, for example, *E. coli*. The isolating of the plasmid DNA may include isolating a cell layer from the culture and isolating the plasmid DNA from the cell layer. The isolating of the cell layer may be performed by centrifugation, separation, precipitation, or a combination thereof. Isolation of plasmid DNA from cells may be performed by methods known in the art, such as an alkali extraction method, affinity chromatography, high performance liquid chromatography (HPLC), electrophoresis, or a combination thereof.

The obtaining of RNA may include producing an RNA transcript of the DNA by culturing the cell including the DNA.

**Advantageous Effects of Invention**

The mRNA according to an aspect is relatively stable and has a high production yield, and thus may be used to transcribe and translate an mRNA efficiently.

When a method of obtaining polypeptide according to another aspect is used, a polypeptide may be obtained efficiently.

The composition for delivering the mRNA or a product expressed thereby to a subject according to another aspect may be used to deliver the mRNA or a product expressed thereby to a subject.

The composition for inducing a therapeutic response or an immune response to a polypeptide according to another aspect may be used to induce a therapeutic response or an immune response to a polypeptide in a subject.

The DNA according to another aspect may be used to obtain the mRNA.

According to the method of obtaining RNA in accordance with another aspect, RNA may be obtained efficiently.

**Best Mode for Carrying out the Invention**

Hereinafter, the present disclosure will be described in more detail through examples. However, these examples are intended to illustrate the present disclosure, and the scope of the present disclosure is not limited to these examples.

**Example 1: Specifically-structured 3'UTR non-natural sequence increasing yield of genes**

Non-natural 3'UTR sequences that form a structure similar to the natural 3'UTR structure having excellent activity in increasing the yield of genes and that have activity in increasing the yield of genes compared to the native 3'UTR structure were selected.

As the native 3'UTR, a human complement component 3 (C3) 3'UTR (SEQ ID NO: 9) or a human cytochrome P450 2E1 3'UTR (SEQ ID NO: 10), which is known to have an excellent activity of increasing the yield of genes, was used. Specifically, random sequence combinations having the same length as the human C3 3'UTR or the human cytochrome P450 2E1 3'UTR were constructed, and sequences of which secondary structures were similar to the human C3 3'UTR or the cytochrome P450 2E1 3'UTR were selected.

Regarding the selected mRNA sequences, a process of removing potential AU-rich element (hereinafter referred to as 'ARE') sequences that can cause mRNA unstable was performed, and then, 8 non-natural 3'UTR sequences were finally selected (Table 1).

Native human C3 3'UTR sequence (SEQ ID NO: 9-length 178 nt):

CCACACCCCCAUUCCCCCACUCCAGAUAAAGCUUCAGUUAUAUCUCACGU
GUCUGGAGUUCUUUGCCAAGAGGGAGAGGCUGAAAUCCCCAGCCGCCUCACCU
GCAGCUCAGCUCCAUCCUACUUGAAACCUCACCUGUUCCCACCGCAUUUUCUCC
UGGCGUUCGCCUGCUAGUGUG

Secondary structure of native C3 3'UTR sequence (represented by dot-bracket notation):

.

(((((...........(((...(((((.(((....))).)))))...)))..(((((((..((((....((((((.(((((.......)))))..))-
).))))))...)))) )))........(((....)))............(((.........(((....))))))..))))

The non-natural sequences (SEQ ID NOs: 1 to 5) having a structure similar to the structure of the native C3 sequences were designed to have a GC-content (GC%) of 40 to 50 % and a mean free energy for RNA folding of -0.1 to -0.3 kcal/mol.

Native human cytochrome P450 2E1 3'UTR sequence (SEQ ID NO: 10-length 158 nt):

GUGUGUGGAGGACACCCUGAACCCCCCGCUUUCAAACAAGUUUUCAAAU UGUUUGAGGUCAGGAUUUCUCAAACUGAUUCCUUUCUUUGCAUAUGAGUAUUU GAAAAUAAAUAUUUUCCCAGAAUAUAAAUAAAUCAUCACAUGAUUAUUUUAACUA U

Secondary structure of native cytochrome P450 2E1 3'UTR sequence: ((((((((((((...(((((.((........((((((((.........)))))))))))))))).....(((...))).....))))))))))))..((((((((((((....) )))))...)))))).....((((((...)))))).........

The non-natural sequences (SEQ ID NOs: 6 to 8) having a structure similar to the structure of the native cytochrome P450 2E1 3'UTR sequence were designed to have a GC% of 30 to 40 % and a mean free energy for RNA folding of -0.1 to -0.3 kcal/mol.

[Table 1]

| SEQ ID NO: | 3'UTR sequence |
|---|---|
| 1 | ACCACCAAGACAAAAACCACGAAUUUACGCCAAAAGUGGAGUAAGGAGG UGCGUCCCUUACGCAACACGACAGCCGGGAUCUAACAAUAGGGUUAAU GGACUGGUUGAGAUAAGGGGCAUUAAAACCAAACGGUCUACACAUAAAA CGAAUGAAAACUUCCUAAAGGAUCGACGGUGG |
| 2 | CAUACCAUAAAACCAAACAUAUACCCGAGGUCCUUCGGCAUUGGGAGGA UGAUACGCAAGAAGCAUGAUUCCCCACCAGACCGAAUGCAGGUUUAAG UGAGGGGUGCAAACUUGUGUCACUAACCAUUAUUAUGAAGAAAAAAACA GCCAAAAAAAGAGCCACAAGGCGGCGCGGUAU |
| 3 | GCCGCUAAAUAAACGAACACGUACCUACGCUUUGACAGGAGUGGGCAA GUGGGAAGGUUACCGGAACUACGAGGACCACCAGGCAAGUAUUGGUUA GUCACUCUUCCAACUAAUCUUAAUAAAACGAGACAUCGAAAAAAAAAAA GGCAAAAACAAGUCCAGAAGGAGCCAGGGUGG |
| 4 | AGAGUAAAAUUAAUAUACGGUAUAGAACUCACCAUUGUGCGUUCUACCU CGAGGUGUCAUGACGACAGUGUCCCAUAUCAACCCGAGAAGUUGGAAA UGCGGAGUCGAACGUGACGCCAAUAAGGCCAAAAGGUAAAUAAUGAAAC GUACAACCGAAUGCCAAAAGGCUACGCUGCUC |
| 5 | GUACAUGCUACAAACCAUGCGGAACCUACAAGACACUUUAUGGGUAAAG CAACAAACCCACUUGUAAAGUUACCACACCGGUCCCAUCUGCCGGCCG UGAGUAUAUGAUCUGGGUUUAUAAUCUCGAAAUAUCGCGAAACAAUAUG CCCUUAGAAUAAGCACCCAUGCGGGCAAUGUA |

(continued)

| SEQ ID NO: | 3'UTR sequence |
|---|---|
| 6 | UAGUAAUACAGCAUAGUGACGCAAAAUUAUUACGAGUACAAUUAAUCAG UACUUGUUGGUCAUAGCAACCAAAGUGGAAAAAGUUGUGUUAUUAAAAC CCCAAAACAAACGAUUGUUUCAAUGGGGUCAUAACUAAUGUAACGUUAG GUAAUUAUCCG |
| 7 | UCUUUAUGAGAAUUACUCAUUCUGAUUUUUAACAUAGCAUUUUUUUUCU GUUGUGUAGAUGGGCCAAAGGACAUUCCAUAACUUUUCAUAAGGACAAA CGAUUACCGACGGAUUGGUACUAGUUGUUAAUUAACAUCUGGGGGAUG UAAACAUUUUUU |
| 8 | AACACAGAAAACUACCACUAUACUUCUAUUUAGAAAGCACUUAUUAAUU GCUUUUUGUUAGUGAAACAUGCAAGGCACCCAUGUUUUCUGUGUUAAC CAAGCCAGUUGAUAUUAGCUGUAUGCUUGGAUUCUAUACAUAAAAUGUA UUUAAGCAGCUC |

The RNA folding energy provides insights into the secondary structure and stability thereof. For example, the minimum free energy may be used to predict the secondary structure of 3'UTR and stability thereof, which may affect the translation efficacy.

**Example 2: Preparation of template vector using non-natural 3'UTR sequence**

One of the sequences encoding the non-natural 3'UTR nucleotide sequences including the nucleotide sequences of SEQ ID NOs: 1 to 8 obtained in Example 1, a sequence encoding the native C3 3'UTR nucleotide sequence, and a gene encoding a polypeptide were operatively linked to one another to prepare a vector. The polypeptide may be a wild-type p53 protein having an amino acid sequence of SEQ ID NO: 11.

Specifically, the vector is one in which a promoter, a nucleotide sequence encoding 5'-UTR, an open reading frame (ORF) encoding a polypeptide, a nucleotide sequence encoding 3'-UTR, and a poly (A) tail.

The vector may also include *E. coli* replication origin ColE1 ori and a selection marker gene. The promoter may be a T7 promoter. The selection marker may be a kanamycin resistance enzyme.

**2-1. Preparation of template vector for transcription of p53 mRNA**

A vector for producing a native p53 mRNA and/or a protein product thereof was prepared, the vector including a nucleotide sequence encoding a native C3 5'UTR sequence, a nucleotide sequence, as an ORF, encoding a native p53 protein, a nucleotide sequence encoding one of the designed non-natural 3'UTR of any one of SEQ ID NOs: 1 to 8, and a native p53 mRNA including poly(A). This vector was then introduced to a microbial cell, and the resulting microbial cell was cultured to produce a cloning vector. Through *in vitro* transcription with the produced vector as a template, mRNA was produced.

Specifically, a vector pUC57-KanR, in which an antibiotic resistance gene was substituted with a kanamycin resistance gene, was produced from the pUC57-AmpR vector (Addgene). The produced vector was treated and cleaved with restriction enzymes HindIII and EcoRI, and the cleaved vector was connected to a synthetically produced polynucleotide encoding a native p53 protein by using a ligase to insert a sequence encoding a native p53 protein. Since the inserted polynucleotide encoding a native p53 protein includes a nucleotide sequence encoding 5'UTR and a sequence encoding a native p53 protein, and restriction enzyme recognition sequences are at the 3'-end of the native p53 nucleotide sequence, the restriction enzyme recognition sequences were cleaved to introduce a nucleotide sequence encoding non-natural 3'UTR.

[Table 2]

| SEQ ID NO: | Gene sequence (DNA) of template vector for transcription of native p53 mRNA |
|---|---|
| 12 | TAATACGACTCACTATA |
| 13 | **AGTCCTCCCCATCCTCTCCCTCTGTCCCTCTGTCCCTCTGACCCTGC ACTGTCCCAGCACC** |
| 14 | ATGGAAGAACCTCAGTCCGATCCGAGCGTCGAGCCTCCCCTGTCACA GGAAACTTTCAGTGACCTGTGGAAACTGCTGCCCGAGAATAATGTACT TTCGCCTCTGCCTAGCCAAGCAATGGATGATCTGATGCTGTCTCCAGA TGACATCGAACAGTGGTTTACAGAGGATCCAGGGCCCGATGAGGCAC CACGAATGCCAGAGGCGGCCCCACCTGTTGCACCCGCACCTGCGGC TCCAACACCCGCAGCCCCCGCCCCCGCTCCAAGCTGGCCTCTCAGTA GTTCCGTGCCATCACAGAAGACCTATCAAGGCAGCTACGGATTTCGC CTCGGATTTCTCCATTCAGGCACTGCCAAGTCCGTGACTTGTACGTAC AGCCCCGCTCTGAACAAAATGTTCTGTCAGTTGGCGAAGACATGTCCT GTCCAGCTTTGGGTAGATTCTACCCCCCCGCCGGGGACACGGGTGC GAGCCATGGCCATTTATAAACAGTCTCAGCACATGACCGAGGTCGTG CGCAGGTGTCCTCACCATGAACGATGCTCCGACTCCGACGGCCTGG CCCCCCCACAGCACCTGATTCGCGTGGAAGGCAATCTTCGTGTGGAG TACCTCGATGATCGGAACACCTTCAGACATAGCGTGGTTGTGCCTTAT GAGCCACCCGAAGTTGGAAGCGACTGTACCACCATCCATTACAACTA TATGTGCAACAGTAGCTGCATGGGTGGCATGAATCGGCGGCCTATCT TGACAATAATCACTTTAGAGGACTCTTCCGGAAACCTGCTTGGCAGAA ATTCATTCGAGGTCCGTGTCTGCGCCTGCCCGGGCAGGGACCGCAG AACCGAAGAGGAGAATTTACGGAAAAAGGGGGAACCCCACCACGAAC TGCCACCGGGCAGTACCAAAAGGGCTCTCCCTAATAACACATCTTCAT CGCCCCAGCCAAAGAAGAAACCCCTCGACGGAGAGTACTTTACTCTA CAAATTAGGGGGAGAGAAAGATTTGAGATGTTCCGCGAATTGAACGA GGCCCTGGAGTTGAAAGACGCTCAAGCCGGAAAGGAACCAGGTGGG TCTAGGGCCCACTCAAGCCATCTGAAGAGTAAGAAAGGCCAGTCCAC TTCCAGGCACAAAAAGTTAATGTTCAAGACGGAGGGTCCTGACTCTGA C |
| 15 | GTGTGTGGAGGACACCCTGAACCCCCCGCTTTCAAACAAGTTTTCAAA TTGTTTGAGGTCAGGATTTCTCAAACTGATTCCTTTCTTTGCATATGAG TATTTGAAAATAAATATTTTCCCAGAATATAAATAAATCATCACATGATT ATTTTAACTAT |

(continued)

| SEQ ID NO: | Gene sequence (DNA) of template vector for transcription of native p53 mRNA |
|---|---|
| 16 | AAAAAAAAAAAAAAAAAAAAAAAAAAAAAAAAAAAAAAAAAAAAAAAAAA AAAAAAAAAAAAAAAAAAAAAAAAAAAAAAAAAAAAAAAAAAAAAAAAAA AA |

Table 2 shows a promoter (SEQ ID NO: 12), a polynucleotide encoding 5'UTR (SEQ ID NO: 13), a polynucleotide encoding a native p53 protein (SEQ ID NO: 14), a polynucleotide encoding 3'UTR (SEQ ID NO: 15), and a poly (A) sequence (SEQ ID NO: 16), included in the prepared pUC57-KanR vector. In Table 2, the underlined nucleotides in SEQ ID NO: 12 represent a portion of a T7 promoter involved in the initiation of mRNA transcription. Also, the bolded nucleotides of SEQ ID NO: 13 represent a human C3 native 5'UTR sequence. Also, SEQ ID NO: 14 is a nucleotide sequence downstream of the 5'UTR, and is a nucleotide sequence encoding a native p53 protein. The native p53 protein had an amino acid sequence of SEQ ID NO: 11. Also, SEQ ID NO: 15 is a native 3'UTR sequence of human cytochrome P450 2E1 gene.

**2-2. Connection of non-natural 3'UTR sequence to nucleotide sequence encoding native p53 protein and cloning**

To substitute the 3'UTR sequence of the prepared template vector with a nucleotide sequence encoding a non-natural 3'UTR sequence, cloning was performed. The 3'UTR may include XhoI and NheI restriction enzyme sequences at the upstream 5'-end and the downstream 3'-end, respectively, to be used for recombination of a nucleic acid such as the transcribable nucleic acid sequence or a poly(A) sequence. The transcribable nucleic acid sequence may be an ORF. The 3'UTR sequence may be linked to the 5'-end present in the sequence.

The recombinant 3'UTR sequence including the XhoI restriction enzyme sequence, the non-natural 3'UTR sequence, and the NheI restriction enzyme sequence was synthesized by an in vitro nucleic acid synthesis method. Each of the recombinant 3'UTR sequence and the template vector was treated and cleaved with restriction enzymes XhoI and NheI, and both the cleaved sequences were connected by using a ligase. For use as the template vector, the vector prepared in Example 2-1 was used.

As a result, a vector in which a T7 promoter-5'UTR-coding nucleotide sequence-p53 protein-coding sequence-nucleotide sequence encoding one of any one of non-natural 3'UTR sequences of SEQ ID NOs: 1 to 8-poly (A) structure was introduced into the backbone of the pUC57-KanR vector was prepared.

**Example 3: Production and yield of gene product using non-natural 3'UTR sequence**

The vector prepared in Example 2, having a 5'UTR sequence-p53 protein-coding sequence-non-natural 3'UTR sequence-poly(A) structure, was introduced into *E. coli*, and the *E. coli* was cultured to proliferate the *E. coli* including a plasmid DNA as a template for the mRNA. The proliferated *E. coli* was isolated from the culture by high-speed centrifugation, and the cell-derived template DNA was secured by using an alkali extraction method. The secured template DNA was linearized by using the restriction enzyme recognition sequence located at the end of the poly (A) tail. Accordingly, an mRNA having a nucleotide sequence with a 5'UTR sequence-p53 protein-coding sequence-non-natural 3'UTR sequence-poly (A) structure was produced via in vitro transcription using the linearized cell-derived template DNA. Here, the 5'UTR has a native C3 sequence (SEQ ID NO: 17), the non-natural 3'UTR has a sequence of one of SEQ ID NOs: 1 to 8, and the poly(A) and the p53 protein-coding protein are as described above.

Specifically, to an aqueous solution containing 10 ng/$\mu$l of the linearized plasmid DNA, 4 mM of a capping reagent, 5 mM of ATP, 5 mM of CTP, 5 mM of GTP, 5 mM of N1-methylpseudouridine-5'-triphosphate, 20 mM of magnesium ion salt, 10 mM of DTT, 0.01 U/$\mu$L of inorganic pyrophosphatase (PPase), and 1 U/$\mu$L of RNAase inhibitor, 5 U/$\mu$L of T7 RNA polymerase was added, and the reaction mixture thus obtained was incubated at 37 °C for at least one hour, to proceed in vitro transcription. For use as the capping reagent, the cap analog compound m7G(3'OMs)pppA(2'OMe)pG of Example 8 described in 10-2023-0141482 published on October 10, 2023 was used. The capping reagent is a reagent for mRNA transcription and co-transcriptional capping, and forms a 5'cap structure of m7G(3'OMs)pppA(2'OMe)pG. Here, m7G represents 7-methylguanosine, A represents adenosine, G represents guanosine, p is -P(=O)(OH)O-, Ms represents mesyl, and Me represents methyl. The reaction product was treated with a DNase, and mRNAs (SEQ ID NOs: 18 to 26) of Materials 1 to 9 were obtained by using a Monarch® RNA Cleanup Kit (New England Biolabs). A configuration of nucleotide sequences of the 5'UTR, p53 protein sequence, and non-natural 3'UTR included in the mRNAs and a relative synthesis yield according to the UTR combination are as shown in Table 3. The mRNAs may further have a 5'-cap structure and a poly (A) sequence structure (SEQ ID NOs: 27 to 35). The relative synthesis yield represents a relative synthesis yield

of the mRNA according to in vitro transcription, and was calculated based on a synthesis yield of Material 1 (100 %).

Relative synthesis yield (%) = (synthesis yield of each material / synthesis yield of Material 1) $\times$ 100

[Table 3]

| Mater ial No. | 5'UTR sequence | ORF sequence | 3'UTR sequence | Yield (mg/mL) | Relative synthesis yield (%) |
|---|---|---|---|---|---|
| 1 | Native C3 | Native p53 | Native CYP2E1 | 6.88 | 100.0 |
| 2 | Native C3 | Native p53 | SEQ ID NO: 1 | 7.20 | 104.7 |
| 3 | Native C3 | Native p53 | SEQ ID NO: 2 | 7.48 | 108.7 |
| 4 | Native C3 | Native p53 | SEQ ID NO: 3 | 6.96 | 101.2 |
| 5 | Native C3 | Native p53 | SEQ ID NO: 4 | 7.44 | 108.1 |
| 6 | Native C3 | Native p53 | SEQ ID NO: 5 | 7.00 | 101.8 |
| 7 | Native C3 | Native p53 | SEQ ID NO: 6 | 7.36 | 107.0 |
| 8 | Native C3 | Native p53 | SEQ ID NO: 7 | 7.32 | 106.4 |
| 9 | Native C3 | Native p53 | SEQ ID NO: 8 | 7.80 | 113.4 |

As shown in Table 3, it is confirmed that the structural changes according to the UTR sequences can lead to different transcription efficiency, which in turn leads to an improved synthesis yield of p53 mRNA through in vitro transcription (IVT) depending on the non-natural 3'UTR. Also, it was confirmed that the translation yield increased in the translation using Materials 2 to 9.

## Claims

1. A messenger RNA (mRNA) comprising a non-natural 3' untranslated region (3'UTR),

   wherein the 3'UTR has: a secondary structure represented by dot-bracket notation below:

   .

   (((((...........(((...(((((.(((....))).)))))...)))..(((((((..((((....((((((.(((((.......)))))..))-
   ).)))) )))...)))))))......(((....)))...........(((.........(((....))))))..)))); or
   a secondary structure represented by dot-bracket notation below:
   (((((((((((...(((((.((........((((((((.........))))))))))))))).....(((...))).....)))))))))))..(-
   (((((( (((((....))))))...)))))).....((((((...))))))..........,
   wherein a dot denotes unpaired bases in a sequence, and "(" and ")" denote paired bases in a sequence.

2. The mRNA of claim 1, wherein the mRNA comprising the 3'UTR has a mean free energy of -0.1 to -0.3 kcal/mol for RNA folding,

   (a) the secondary structure of the mRNA comprising the 3'UTR, represented by

   .

   (((((...........(((...(((((.(((....))).)))))...)))..(((((((..((((....((((((.(((((.......)))))..))-
   ).)))) )))...)))))))......(((....)))...........(((.........(((....))))))..))))), has a length of 178 nt and a GC-content (GC %) of 40 to 50 %, and
   (b) the secondary structure of the mRNA comprising the 3'UTR, represented by
   (((((((((((...(((((.((........((((((((.........))))))))))))))).....(((...))).....)))))))))))..(-
   (((((( (((((....))))))...)))))).....((((((...)))))).........., has a length of 158 nt and a GC% of 30 to 50 %.

3. The mRNA of claim 2, wherein the mRNA comprising the 3'UTR comprises a sequence selected from the group consisting of nucleotide sequences of SEQ ID NOs: 1 to 8.

4. The mRNA of claim 1, wherein the mRNA further comprises a sequence encoding a polypeptide.

**5.** The mRNA of claim 4, wherein the mRNA further comprises a Kozak sequence, a 5'UTR sequence, or a poly (A) sequence.

**6.** The mRNA of claim 1, wherein the mRNA comprises a 5'-cap selected from Cap0, Cap1, and Cap2.

**7.** The mRNA of claim 1, wherein the mRNA has one or more modified nucleotides.

**8.** DNA encoding the mRNA of any one of claims 1 to 7.

**9.** A composition comprising, as an active ingredient, the mRNA or DNA of any one of claims 1 to 8.

## INTERNATIONAL SEARCH REPORT

International application No.
**PCT/KR2024/014734**

**A. CLASSIFICATION OF SUBJECT MATTER**

**C12N 15/11**(2006.01)i; **C12N 15/113**(2010.01)i; **C12N 15/63**(2006.01)i

According to International Patent Classification (IPC) or to both national classification and IPC

**B. FIELDS SEARCHED**

Minimum documentation searched (classification system followed by classification symbols)

C12N 15/11(2006.01); C12N 15/113(2010.01); C12N 15/67(2006.01); C12N 9/02(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 3'비번역 영역(3'UTR, 3' untranslated region), mRNA, 2차 구조(secondary structure), 5'UTR, 폴리 A(poly A), 5'캡(5'CAP)

**C. DOCUMENTS CONSIDERED TO BE RELEVANT**

| Category* | Citation of document, with indication, where appropriate, of the relevant passages | Relevant to claim No. |
|---|---|---|
| X<br>Y<br>A | TANTS, J.-N. et al. NMR-derived secondary structure of the full-length Ox40 mRNA 3`UTR and its multivalent binding to the immunoregulatory RBP Roquin. Nucleic Acids Research. electronic publication on 31 March 2022, vol. 50, no. 7, pp. 4083-4099.<br>See abstract; page 4084, right column, page 4087, left column and page 4088, left column; and figure 1. | 1-2,4,7-8<br>5-6<br>3 |
| Y | KR 10-2023-0083893 A (KOREA UNIVERSITY RESEARCH AND BUSINESS FOUNDATION et al.) 12 June 2023 (2023-06-12)<br>See claims 1-6 and 11-14. | 5-6 |
| Y | KR 10-2023-0000471 A (HANMI PHARM. CO., LTD.) 02 January 2023 (2023-01-02)<br>See claims 1-26; and paragraphs [0039] and [0056]. | 5-6 |
| A | US 2019-0071682 A1 (BIONTECH RNA PHARMACEUTICALS GMBH et al.) 07 March 2019 (2019-03-07)<br>See entire document. | 1-8 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **09 January 2025** | **09 January 2025** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.
**PCT/KR2024/014734**

| **C. DOCUMENTS CONSIDERED TO BE RELEVANT** | | |
|---|---|---|
| Category* | Citation of document, with indication, where appropriate, of the relevant passages | Relevant to claim No. |
| A | KR 10-2017-0083172 A (EWHA UNIVERSITY - INDUSTRY COLLABORATION FOUNDATION) 18 July 2017 (2017-07-18)<br>See entire document. | 1-8 |

Form PCT/ISA/210 (second sheet) (July 2022)

**INTERNATIONAL SEARCH REPORT**

International application No.
**PCT/KR2024/014734**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/KR2024/014734**

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☑ Claims Nos.: **9**
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/KR2024/014734**

| Patent document cited in search report | Publication date (day/month/year) | Patent family member(s) | Publication date (day/month/year) |
|---|---|---|---|
| KR 10-2023-0083893 A | 12 June 2023 | AR 127850 A1 | 06 March 2024 |
| | | AU 2022-401495 A1 | 20 June 2024 |
| | | CA 3239776 A1 | 08 June 2023 |
| | | CN 118339293 A | 12 July 2024 |
| | | EP 4441222 A1 | 09 October 2024 |
| | | JP 2024-545435 A | 06 December 2024 |
| | | TW 202323527 A | 16 June 2023 |
| | | WO 2023-101508 A1 | 08 June 2023 |
| KR 10-2023-0000471 A | 02 January 2023 | AR 126239 A1 | 04 October 2023 |
| | | AU 2022-297171 A1 | 18 January 2024 |
| | | CA 3223855 A1 | 29 December 2022 |
| | | CN 117597446 A | 23 February 2024 |
| | | EP 4361270 A1 | 01 May 2024 |
| | | IL 309530 A | 01 February 2024 |
| | | JP 2024-528447 A | 30 July 2024 |
| | | MX 2023015487 A | 10 April 2024 |
| | | TW 202317766 A | 01 May 2023 |
| | | US 2024-0301433 A1 | 12 September 2024 |
| | | WO 2022-270969 A1 | 29 December 2022 |
| US 2019-0071682 A1 | 07 March 2019 | AU 2016-335029 A1 | 19 April 2018 |
| | | AU 2016-335029 B2 | 29 April 2021 |
| | | AU 2021-206886 A1 | 12 August 2021 |
| | | AU 2021-206886 B2 | 14 December 2023 |
| | | AU 2023-278107 A1 | 04 January 2024 |
| | | BR 112018003486 A2 | 25 September 2018 |
| | | BR 112018003486 B1 | 26 September 2023 |
| | | BR 122021025194 A2 | 25 September 2018 |
| | | CA 2998554 A1 | 13 April 2017 |
| | | CA 2998554 C | 30 April 2024 |
| | | CA 3223575 A1 | 13 April 2017 |
| | | CN 108291230 A | 17 July 2018 |
| | | CN 108291230 B | 20 December 2022 |
| | | CY 1122347 T1 | 27 January 2021 |
| | | CY 1124066 T1 | 27 May 2022 |
| | | DK 3337902 T3 | 04 November 2019 |
| | | DK 3636764 T3 | 22 March 2021 |
| | | EP 3337902 A2 | 27 June 2018 |
| | | EP 3337902 B1 | 25 September 2019 |
| | | EP 3636764 A1 | 15 April 2020 |
| | | EP 3636764 B1 | 03 March 2021 |
| | | EP 3868885 A1 | 25 August 2021 |
| | | ES 2753201 T3 | 07 April 2020 |
| | | ES 2753201 T8 | 16 April 2020 |
| | | ES 2866224 T3 | 19 October 2021 |
| | | HK 1256701 A1 | 04 October 2019 |
| | | HK 1258262 A1 | 08 November 2019 |
| | | IL 258074 A | 31 May 2018 |
| | | IL 258074 B | 29 July 2021 |
| | | IL 284651 A | 31 August 2021 |
| | | IL 284651 B | 01 June 2022 |

Form PCT/ISA/210 (patent family annex) (July 2022)

**INTERNATIONAL SEARCH REPORT**
**Information on patent family members**

International application No.
**PCT/KR2024/014734**

| Patent document cited in search report | Publication date (day/month/year) | Patent family member(s) | Publication date (day/month/year) |
|---|---|---|---|
| | | IL 293020 A | 01 July 2022 |
| | | JP 2018-530332 A | 18 October 2018 |
| | | JP 2021-048847 A | 01 April 2021 |
| | | JP 2021-048848 A | 01 April 2021 |
| | | JP 2021-129563 A | 09 September 2021 |
| | | JP 2022-050390 A | 30 March 2022 |
| | | JP 2024-138270 A | 08 October 2024 |
| | | JP 6844060 B1 | 17 March 2021 |
| | | JP 7084302 B2 | 14 June 2022 |
| | | JP 7084565 B2 | 15 June 2022 |
| | | KR 10-2018-0057647 A | 30 May 2018 |
| | | KR 10-2022-0025195 A | 03 March 2022 |
| | | KR 10-2024-0118189 A | 02 August 2024 |
| | | KR 10-2363368 B1 | 16 February 2022 |
| | | KR 10-2688535 B1 | 26 July 2024 |
| | | LT 3337902 T | 10 December 2019 |
| | | LT 3636764 T | 10 May 2021 |
| | | MX 2018004160 A | 01 August 2018 |
| | | MX 2021000250 A | 12 April 2021 |
| | | NZ 740946 A | 26 August 2022 |
| | | PL 3337902 T3 | 31 January 2020 |
| | | PL 3636764 T3 | 05 July 2021 |
| | | PT 3337902 T | 30 October 2019 |
| | | PT 3636764 T | 25 March 2021 |
| | | RS 59538 B1 | 31 December 2019 |
| | | RS 61684 B1 | 31 May 2021 |
| | | RU 2018112325 A | 07 November 2019 |
| | | SG 10201912558 A | 27 February 2020 |
| | | SI 3337902 T1 | 31 December 2019 |
| | | SI 3636764 T1 | 30 July 2021 |
| | | US 11492628 B2 | 08 November 2022 |
| | | US 2023-0272406 A1 | 31 August 2023 |
| | | WO 2017-059902 A1 | 13 April 2017 |
| | | WO 2017-060314 A2 | 13 April 2017 |
| | | WO 2017-060314 A3 | 18 May 2017 |
| | | ZA 201800835 B | 27 May 2020 |
| KR 10-2017-0083172 A | 18 July 2017 | KR 10-1856308 B1 | 10 May 2018 |
| | | KR 10-2017-0104015 A | 14 September 2017 |
| | | WO 2017-119576 A1 | 13 July 2017 |

Form PCT/ISA/210 (patent family annex) (July 2022)